# EUROPÄISCHE PATENTSCHRIFT

(11) **EP 1 584 607 B1**
(45) Veröffentlichungstag und Bekanntmachung des Hinweises auf die Patenterteilung: **16.04.2008**
(21) Anmeldenummer: 05003561.7
(22) Anmeldetag: 18.02.2005
(51) Int. Cl.: C03C 10/04, C03C 10/10, A61K 6/06

(54) **Apatitglaskeramik auf der Basis von silicatischen Oxyapatiten**
Apatite glass ceramics on the basis of siliceous oxyapatites
Vitrocéramique d'apatite à base d'oxyapatites silicieux

(30) Priorität: 18.03.2004 DE 102004013455
(43) Veröffentlichungstag der Anmeldung: 12.10.2005
(73) Patentinhaber: Ivoclar Vivadent AG, FL-9494 Schaan (LI)
(72) Erfinder: Apel, Elke, 9470 Buchs (CH); Höland, Wolfram, FL-9494 Schaan (LI); Ritzberger, Christian, 6710 Nenzing (AT); Bolle, Urs, 6800 Feldkirch (AT); Rheinberger, Volker M., 9490 Vaduz (LI)
(74) Vertreter: UEXKÜLL & STOLBERG

(56) Entgegenhaltungen:
- UO M ET AL: "THE EFFECT OF RARE-EARTH OXIDES ON THE CRYSTALLIZATION OF CAO-AL2O3-SIO2 GLASSES" JOURNAL OF MATERIALS SCIENCE, CHAPMAN AND HALL LTD. LONDON, GB, Bd. 33, Nr. 3, 1. Februar 1998 (1998-02-01), Seiten 749-754, XP000733026 ISSN: 0022-2461

## Beschreibung

Die Erfindung betrifft Apatitglaskeramiken, die als kristalline Phase silicatischen Oxyapatit enthalten. Diese Glaskeramiken eignen sich auf Grund ihrer physikalischen und chemischen Eigenschaften besonders zur Verwendung in der Dentaltechnik.

Apatit ist ein phosphathaltiges Mineral mit der allgemeinen Formel Ca₁₀ (PO₄)₆ (OH, F, Cl)₂. Hydroxyapatit, Ca₅ (PO₄)₃ OH, ist der Hauptbestandteil des Zahnschmelzes der Zähne und der festen Substanz der Knochen, wobei auch Anteile von CO₃²⁻ enthalten sind. In der wissenschaftlichen Fachliteratur der Kristallographie werden aber auch, z. B. in Jun Ito, The American Mineralogist 53 (1968) 890, Apatite beschrieben, die phosphat- und fluorfrei sind. Dies ist zunächst ungewöhnlich, kann aber mit Isotypie-oder sogar Isomorphiebeziehungen zwischen Phosphaten und Silikaten erklärt werden. Diese phosphatfreien Apatite werden als silicatische Oxyapatite bezeichnet.

Bisher sind keine Glaskeramiken beschrieben worden, die phosphat- und fluorfreie silicatische Oxyapatitkristalle enthalten. Es sind bisher nur stofflich verwandte apatithaltige Glaskeramiken bekannt, die im Folgenden vorgestellt werden.

In der Zahnmedizin wurden für den restaurativen Zahnersatz, zur Herstellung von Inlays, Kronen, Brücken und Verblendschalen, leucithaltige Glaskeramiken entwickelt, die nadelförmige Apatitkristalle enthalten können.

Die DE 44 23 793 offenbart leucithaltige Phosphosilikat-Glaskeramiken, die neben einer Leucit-Kristallphase mindestens eine weitere Kristallphase und eine oder mehrere Glasphasen enthalten. Bevorzugt sind Glaskeramiken, die als weitere Kristallphase phosphathaltige, nadelförmige Apatitkristalle enthalten.

Aus der US 5,952,253 sind transparente Glaskeramiken bekannt, die als kristalline Phase Apatitkristalle enthalten. Die Glaskeramiken weisen einen relativ hohen Al₂O₃-Gehalt und weniger als 20 Vol.-% Apatitkristalle auf. Diese Apatitkristalle können Silicat enthalten, besitzen aber keine Sauerstoffionen in der (OH,F,Cl)-Position und sind somit nicht als silicatische Oxyapatite zu bezeichnen. Die Glaskeramiken sollen sich insbesondere für polarisationssensitive Anwendungen, wie Vergrößerungslinsen und Laser, eignen. Sie eignen sich auf Grund ihrer vollständigen optischen Transparenz nicht für dentale Anwendungen.

Die DE 103 40 597 betrifft transluzente, radioopake Phosphosilicat-Glaskeramiken mit verbesserten optischen Eigenschaften, insbesondere mit einer gezielt einstellbaren Transluzenz und optischen Helligkeit, die nadelförmige, phosphathaltige Apatitkristalle und Leucitkristalle enthalten. Beide Kristallphasen entstehen aus einem Ausgangsglas ohne Zumischen einer anderen Glaskeramikzusammensetzung. Diese Glaskeramiken zeichneten sich dadurch aus, daß sie optisch den Eigenschaften der natürlichen menschlichen Zähne sehr nahe kommen.

Transluzente Apatitglaskeramiken mit phosphat- und fluorfreien silicatischen Oxyapatitkristallen, deren lineare thermische Ausdehnungskoeffizienten in einem weiten Bereich variiert werden kann und die eine gezielt einstellbare Transluzenz aufweisen, sind somit nach dem Stand der Technik bisher nicht bekannt.

Der Erfindung liegt die Aufgabe zu Grunde, Glaskeramiken zur Verfügung zu stellen, deren linearer thermischer Ausdehnungskoeffizient in einem großen Bereich variiert werden kann, deren optische Eigenschaften gezielt einstellbar sind und die darüber hinaus eine hohe chemische Beständigkeit und gute Verarbeitungseigenschaften auf unterschiedlichsten Substratwerkstoffen aufweisen.

Diese Aufgabe wird durch Apatitglaskeramiken gemäß Anspruch 1 mit mindestens einer Glasphase und mindestens einer Apatitphase gelöst, bei denen mindestens eine der Apatitphasen eine phosphat- und fluorfreie silicatische Oxyapatitphase ist. Durch Variation der Größe und des Anteils der Apatitkristalle läßt sich der Opazitätsgrad der Glaskeramiken einstellen. Bevorzugt sind Glaskeramiken, die 1 bis 8 und insbesondere 1 bis 3 unterschiedliche fluorfreie silicatische Oxyapatitphasen enthalten.

Unter silicatischen Oxyapatiten werden Kristallphasen verstanden, die in der Kristallstruktur des Apatits an Stelle von [PO₄]-Tetraedern [SiO₄]-Tetraeder besitzen und deren (OH,F,Cl) - Positionen durch Sauerstoffionen besetzt sind.

Die Oxyapatitkristalle enthalten damit nicht nur kein Phosphat sondern sind außerdem damit frei von Fluor. Demgegenüber kann die Glasphase der Glaskeramik Fluor und auch Phosphat enthalten.

Die Opazität der erfindungsgemäßen Apatitglaskeramiken kann in einem Bereich von 0,3 bis 0,96, gemessen nach der Norm BS (British Standard) 5612-1978, eingestellt werden. Die Glaskeramiken weisen damit eine Transluzenz und eine Opazität auf, die weitgehend denen der natürlichen Zähne entsprechen.

Bevorzugt sind Apatitglaskeramiken, die als Apatitphase(n) ausschließlich phosphat- und fluorfreie silicatische Oxyapatite enthalten. Unter phosphat- und fluorfreie silicatischen Oxyapatiten werden Silicate der allgemeinen Formel Me[I]ₓMe[II]_{y}Me[III]_{z}(SiO₄)₆O₂ verstanden, wobei Me[I] jeweils für ein einwertiges Kation, Me[II] jeweils für ein zweiwertiges und Me[III] jeweils für ein dreiwertiges Kation steht und x, y und z so gewählt sind, daß die Summe der Wertigkeiten der ein-, zwei-und dreiwertigen Kationen einen Wert von 28 ergibt. Die Indizes x, y und z können den Wert 0 annehmen, es muß aber immer mindestens einer dieser Indizes von 0 verschieden sein. Diese Kristallphasen werden im folgenden der Einfachheit halber auch als silicatische Oxyapatite bezeichnet.

Me[I], Me[II] und Me[III] sind Metallkationen, wobei Me[I] Na oder Li, Me[II] Ca oder Sr und Me[III] Y oder La ist.

Erfindungsgemäß sind besonders solche Apatitglaskeramiken bevorzugt, die eine oder mehreren Oxyapatitphasen der Formel Me[I]Me[III]₉(SiO₄)₆O₂, Me[II]₂Me[III]₈(SiO₄) ₆O₂ und/oder Me [III] _{9,33} (SiO₄) ₆O₂ enthalten, ganz besonders solche, die eine oder mehrere silicatische Oxyapatitphasen der Formel NaY₉(SiO₄)₆O₂, LiY₉(SiO₄)₆O₂, Sr₂Y₈(SiO₄)₆O_{2,} Ca₂Y₈(SiO₄)₆O₂, Sr₂La₈ (SiO₄) ₆O₂ und/oder La_{9,33} (SiO₄) ₆O₂ enthalten.

Neben der oder den silicatischen Oxyapatitkristallphase(n) können die erfindungsgemäßen Apatitglaskeramiken eine oder mehrere, vorzugsweise 1 bis 8, besonders bevorzugt 1 bis 3 weitere Kristallphasen enthalten. Als weitere Kristallphase ist insbesondere Leucit (KAlSi₂O₆) bevorzugt. Besonders bevorzugte erfindungsgemäße Glaskeramiken enthalten eine oder mehrere unterschiedlich zusammengesetzte fluorfreie silicatische Oxyapatitkristallphasen, ganz besonders bevorzugt 1 bis 3 fluorfreie silicatische Oxyapatitkristallphasen, und 1 bis 2 Leucitphasen.

Die erfindungsgemäßen Glaskeramiken enthalten vorzugsweise 1 bis 8, besonders bevorzugt 1 bis 2 Glasphasen.

Der Nachweis der kristallinen Phase erfolgt durch rasterelektronenmikroskopische Aufnahmen (REM) und Raumtemperatur-Röntgenbeugungsanalysen(RT-XRD). Die erfindungsgemäßen Apatitglaskeramiken enthalten vorzugsweise die folgenden Komponenten, wobei sich alle Prozentangaben, wenn nicht anders angegeben, auf die Gesamtmasse der Glaskeramik beziehen:

| | |
|---|---|
| SiO₂ | 40 bis 70 Gew.-% |
| B₂O₃ | 0 bis 9 Gew.-% |
| Al₂O₃ | 7 bis 15 Gew.-% |
| Y₂O₃ | 0 bis 14 Gew.-% |
| La₂O₃ | 0 bis 17 Gew.-% |
| ZrO₂ | 0 bis 3 Gew.-% |
| Li₂O | 0 bis 8 Gew.-% |
| Na₂O | 0 bis 24 Gew.-% |
| K₂O | 0 bis 14 Gew.-% |
| CaO | 0 bis 9 Gew.-% |
| SrO | 0 bis 15 Gew.-% |
| F | 0 bis 3 Gew.-%. |

Für diese Komponenten existieren die folgenden, bevorzugten Mengenbereiche, die unabhängig voneinander gewählt werden können:

| | |
|---|---|
| SiO₂ | 45,9 bis 64,0 Gew.-% |
| B₂O₃ | 0 bis 8,5 Gew.-% |
| Al₂O₃ | 7,9 bis 14,3 Gew.-% |
| Y₂O₃ | 0 bis 12,0 Gew.-% |
| La₂O₃ | 0 bis 14,9 Gew.-% |
| ZrO₂ | 0 bis 1,7 Gew.-% |
| Li₂O | 0 bis 6,0 Gew.-% |
| Na₂O | 0 bis 20,0 Gew.-% |
| K₂O | 0 bis 11,6 Gew.-% |
| CaO | 0 bis 7,3 Gew.-% |
| SrO | 0 bis 12,8 Gew.-% |
| F | 0 bis 2,2 Gew.-%. |

Es sind für Apatitglaskeramiken mit hohem Trübungsgrad Zusammensetzungen bevorzugt, in denen die Summe der Massen der Komponenten Al₂O₃, Y₂O₃ und La₂O₃ 12,9 bis 25,9 Gew.-% bezogen auf die Gesamtmasse der Glaskeramik beträgt.

Weiterhin sind Apatitglaskeramiken bevorzugt, in denen die Summe der Massen der Komponenten Li₂O, Na₂O und K₂O 10,5 bis 20,0 Gew.-% beträgt.

Für die Eigenschaften der Apatitglaskeramik ist es vorteilhaft, wenn der Anteil der Apatitkristalle mindestens 5 Gew.-%, vorzugsweise 10 bis 35 Gew.-% beträgt.

Für die Eigenschaften der Apatitglaskeramik ist es vorteilhaft, wenn der Anteil der weiteren Kristallphasen, bevorzugt Leucit, mehr als 5 Gew.-%, vorzugsweise 10 bis 70 Gew.-% beträgt.

Die Apatitkristalle selbst sind vorzugsweise nadelförmig und haben gemäß einer weiter bevorzugten Ausführungsform eine durchschnittliche Länge von 0,1 bis 10 µm, bezogen auf die Anzahl der Kristalle.

Die Apatitkristalle weisen vorzugsweise ein durchschnittliches Aspektverhältnis von 0,1 bis 100 auf, bezogen auf die Anzahl der Kristalle. Das Aspektverhältnis ist das Verhältnis der mittleren Kristallänge zum mittleren Kristalldurchmesser.

Die erfindungsgemässen Apatitglaskeramiken können als Trübungskomponente vorzugsweise für Sinterglaskeramiken im Dentalbereich eingesetzt werden. Sie sind auch geeignet als Beschichtungs-oder Verblendmaterialien für Substrate, wie Dentalsuprastrukturen auf der Basis von keramischen und glaskeramischen Werkstoffen. Sie weisen einen einstellbaren linearen thermischen Ausdehnungskoeffizienten (WAK) auf und sind daher für unterschiedliche Substratmaterialien geeignet.

Dentale Restaurationen bestehen üblicherweise aus einer Substruktur, auf die in einem oder mehreren Sintervorgängen ein Verblendmaterial aufgebracht wird, das die eigentliche Zahnhartsubstanz ersetzt. Als Gerüstmaterialien kommen eine Reihe von Werkstoffen in Frage, allen voran unterschiedlich zusammengesetzte Metallegierungen, aber auch geformte Werkstücke aus Zirkonoxid ZrO₂, Aluminiumoxid Al₂O₃ sowie hochfesten Glaskeramiken, z.B. auf der Basis von Lithiumdisilikat. Der Umfang dentaler Restaurationen reicht von Einzelkronen bis hin zu großen Arbeiten, die den gesamten Zahnbogen ersetzen können. Welcher Werkstoff bei welcher Restauration zum Tragen kommt, ist im wesentlichen durch die Indikation bestimmt. Die Herstellung künstlichen Zahnersatzes unter Verwendung keramischer Werkstoffe erfordert in der Regel eine Abstimmung der Eigenschaften der Verblendmaterialien auf die Gerüstmaterialien, um für den geplanten Einsatzzweck ausreichende mechanische Eigenschaften zu erhalten.

Auf Grund der breiten Palette an Gerüstmaterialien sind Verblendmaterialien erforderlich, deren Eigenschaften sich über einen weiten Bereich variieren lassen. Ein wichtiger Parameter ist die Sinter- oder Brenntemperatur des Verblendmaterials, die auf das Gerüstmaterial abgestimmt werden muß, um Deformationen infolge einer zu hohen thermischen Belastung zu verhindern. Eine zu tiefe Brenntemperatur kann dagegen zu mangelnder Haftung führen.

Ein weiterer wichtiger Parameter ist der lineare thermische Ausdehnungskoeffizient. Der optimale Verbund zwischen Substruktur und nachfolgend aufgebrannten Schichten wird durch die korrekte Anpassung des linearen thermischen Ausdehnungsverhaltens des Verblendmaterials an das Gerüstmaterial erreicht. Der Bereich für den thermischen Ausdehnungskoeffizienten wird durch Aluminiumoxid Al₂O₃ (WAK ca. 6, 9 * 10⁻⁶ K⁻¹) und hochgoldhaltige Gußlegierungen (WAK ca. 16,0 * 10⁻⁶ K⁻¹) markiert.

Nicht zu vernachlässigen im Hinblick auf das Verbundverhalten ist die Benetzung des Substrats durch das Verblendmaterial bei hohen Temperaturen. Mangelnde Benetzung kann eine Abhebung des Schichtmaterials bewirken, die unter Kaulast zum Versagen der Restauration führt.

Da Dentalwerkstoffe in der Mundhöhle ständig einer großen Vielzahl von chemischen Einflüssen ausgesetzt sind, wie beispielsweise sauren Flüssigkeiten, kommt ihrer chemischen Beständigkeit (nach ISO 9693) eine große Bedeutung zu. Als Maß für die chemische Beständigkeit von Glaskeramiken im Dentalbereich dient die Säurebeständigkeit.

Für den klinischen Einsatz ist darüber hinaus die Biegefestigkeit (nach ISO 6872) maßgeblich.

Der künstliche Ersatz der Zahnhartsubstanz erfordert außerdem die individuelle Anpassung der Restauration an die jeweilige klinische Situation des Patienten. Zu diesem Zweck müssen die unterschiedlichen keramischen Verblendmaterialien über eine definierte Transluzenz (ΔCR) verfügen.

Die erfindungsgemäßen Glaskeramiken erlauben es, die oben genannten Parameter Brenntemperatur, linearer thermischer Ausdehnungskoeffizient (WAK), Benetzung, Chemische Beständigkeit, Biegefestigkeit und Transluzenz durch die Wahl unterschiedlicher Zusammensetzungen und/oder die Variation ihrer thermischen Behandlung gezielt einzustellen.

Die Einstellung des WAK erfolgt durch die gezielte Veränderung der chemischen Zusammensetzung der erfindungsgemäßen Apatitglaskeramik innerhalb der genannten Bereiche und die gezielte Steuerung der Kristallisation. Durch gezielte Variation der leucitbildenden Komponenten K₂O, Al₂O₃ und SiO₂ wird beispielsweise erreicht, daß durch gesteuerte Oberflächenkristallisation Leucitkristalle in der Glaskeramik gebildet werden, die eine Erhöhung des WAKs bewirken. Die Oberflächenkristallisation kann durch Aktivierung der Glasoberfläche durch Feinmahlen und Herstellen eines Glasgranulats beschleunigt werden.

Der lineare thermische Ausdehnungskoeffizient (WAK) der erfindungsgemäßen Apatitglaskeramiken liegt bei Temperaturen von 100 bis 500 °C im Bereich von 6 * 10⁻⁶ K⁻¹ bis 15 * 10⁻⁶ K⁻¹. Die Glaskeramiken eignen sich daher sowohl zur Beschichtung oder Verblendung von hochgoldhaltigen Dentallegierungen mit einem WAK-Bereich von 14 bis 16 * 10⁻⁶ K⁻¹, wie Ag-Au-, Au-, Au-Pt-Legierungen, als auch zur Beschichtung und Verblendung von Al₂O₃-, ZrO₂- Keramiken, Titan oder Titanlegierungen mit WAKs im Bereich von 7 bis 12 * 10⁻⁶ K⁻¹ sowie Ag-Pd-, Pd- und Co-Cr-Legierungen.

Auch die Aufbrenntemperatur der erfindungsgemäßen Apatitglaskeramiken läßt sich in einem weiten Bereich variieren und damit an die unterschiedlichsten Substrate anpassen. Eine Anpassung der Brenntemperatur kann durch die Variation der Zusammensetzung der Glaskeramik oder durch die Verwendung weiterer Gläser oder Glaskeramiken als Mischungskomponente(n) für die erfindungsgemäße Apatitglaskeramik erfolgen. Bevorzugt sind Apatitglaskeramiken, die eine Brenntemperatur von 830 °C bis 1150 °C aufweisen.

Die erfindungsgemäßen Apatitglaskeramiken weisen vorzugsweise eine gemäß der ISO-Vorschrift 9693 gemessene chemische Beständigkeit von < 100 µg/cm², besonders bevorzugt von < 80 µg/cm² und ganz besonders bevorzugt von 10 bis < 80 µg/cm² auf.

Die erfindungsgemäßen Apatitglaskeramiken zeichnen sich außerdem durch eine steuerbare Transluzenz, Trübung und eine hohe Helligkeit aus.

Die Transluzenz läßt sich durch Variation der chemischen Zusammensetzung der Glaskeramik und durch Variation der Bedingungen der thermischen Behandlungen steuern. Ein hohe Transluzenz und damit eine geringe Trübung wird durch die Ausscheidung geringer Kristallmengen erreicht, d.h von Kristallmengen im unteren Bereich der oben definierten Kristallgehalte. Umgekehrt ergeben hohe Gehalte an Kristallen in der Glaskeramik eine hohe Trübung und eine geringe Transluzenz.

Die Herstellung der erfindungsgemäßen Glaskeramiken erfolgt vorzugsweise, indem man
(a) die zur Herstellung der Glaskeramik erforderlichen Ausgangskomponenten mischt und zu einem Glas aufschmilzt, vorzugsweise bei einer Temperatur im Bereich von 1200 bis 1650°C
(b) man die Glasschmelze aus Schritt (a) in ein Glasgranulat überführt, vorzugsweise indem man die Glasschmelze in Wasser gießt,
(c) man das Glasgranulat aus Schritt (b) gegebenenfalls zu einem Glaspulver mit einer mittleren Korngröße von 1 bis 500 µm, bezogen auf die Teilchenanzahl, zerkleinert, und
(d) man anschließend das Glasgranulat aus Schritt (b) oder das Glaspulver aus Schritt (c) einer thermischen Behandlung (ein- oder mehrstufig) im Temperaturbereich von 700 bis zu 1200°C für eine Dauer von 30 Minuten bis 6 Stunden je Behandlungsschritt unterwirft.

In Stufe (a) werden zunächst die erforderlichen Einzelkomponenten, wie z.B. Carbonate, Oxide und Fluoride homogen miteinander vermischt und bei den angeführten Temperaturen aufgeschmolzen. Hierbei bildet sich das gewünschte Ausgangsglas, das anschließend in eine Glaskeramik überführt wird.

Nachfolgend wird in Stufe (b) die erhaltene Glasschmelze durch Eingießen in Wasser abgeschreckt. Es bildet sich ein Glasgranulat. Dieser Herstellungsschritt wird üblicherweise als Fritten bezeichnet.

Gegebenenfalls wird in Stufe (c) das Glasgranulat nachträglich zerkleinert, d.h. vorzugsweise mit üblichen Mühlen auf die bevorzugte Korngröße gemahlen. Das so hergestellte Glaspulver weist vorzugsweise eine mittlere Korngröße von 1 bis 500 µm, bezogen auf die Teilchenanzahl auf.

Dann wird in Stufe (d) das Glasgranulat oder gegebenenfalls das Glaspulver einer thermischen Behandlung bei Temperaturen von 700 bis 1200°C für eine Dauer von 30 Minuten bis 6 Stunden, vorzugsweise 30 Minuten bis 3 Stunden pro Behandlungsschritt unterworfen. Die thermische Behandlung kann in einem oder in mehreren Schritten erfolgen. Vorzugsweise umfaßt sie 1 bis 4, insbesondere 1 oder 2 Schritte. Eine Temperatur von 900°C bis 1200 °C ist bevorzugt, da sie die Ausscheidung der silicatischen Oxyapatitkristalle in der erfindungsgemässen Form und Menge begünstigt.

Ein besonderer Vorteil des erfindungsgemäßen Verfahren ist darin zu sehen, daß erstmals Apatitglaskeramiken zugänglich werden, die phosphat- und fluorfreien silicatischen Oxyapatit enthalten.

Durch rasterelektronenmikroskopische und Röntgenbeugungsuntersuchungen konnte nachgewiesen werden, daß silicatische Oxyapatite die Hauptkristallphase der erfindungsgemäßen Apatitglaskeramiken darstellen. Die Kristallitgröße kann durch unterschiedliche thermische Behandlungen (ein- oder mehrstufig, unterschiedliche Temperaturen) gesteuert werden. Zudem wurde überraschend gefunden, daß durch gezielte Veränderung der chemischen Zusammensetzung und durch Nutzung verschiedener Kristallisationsmechanismen zusätzlich zu den silicatischen Oxyapatitkristallen die Bildung weiterer kristalliner Phasen angeregt werden kann.

Erste systematische Untersuchungen der Wachstumsprozesse der Oxyapatitkristalle deuten darauf hin, daß es sich um einen Oberflächenwachstumsmechanismus handelt. Da z.B. auch Leucit nach dem Mechanismus der Oberflächenkristallisation gebildet wird, findet bei leucithaltigen, erfindungsgemäßen Apatitglaskeramiken eine doppelte, gesteuerte Oberflächenkristallisation statt, die nach dem Stand der Technik bei Glaskeramiken bisher nicht beobachtet wurde.

Zur Weiterverarbeitung der Glaskeramiken werden diese vorzugsweise in Pulverform mit Wasser oder einem geeigneten anderen Lösungsmittel angefeuchtet, geformt und anschließend gebrannt. Gemäß einer ebenfalls bevorzugten, alternativen Verarbeitungsverfahren werden die pulverförmigen Glaskeramiken zunächst zu Formkörpern gesintert, die zur weiteren Formgebung im viskosen Zustand heiß verpreßt oder bei Raumtemperatur durch CAD/CAM-Verfahren mechanisch bearbeitet werden können.

Die erfindungsgemäßen Glaskeramiken zeichnen sich durch eine überraschend guten Mischbarkeit mit anderen Gläsern und/oder Glaskeramiken aus. Aus diesem Grund können die oben genannten Parameter wie Brenntemperatur, linearer thermischer Ausdehnungskoeffizient (WAK), Benetzung, chemische Beständigkeit, Biegefestigkeit Transluzenz und Trübung auch in einfacher Weise durch eine Mischungsreihe eingestellt werden. Hierzu werden eine oder mehrere erfindungsgemäße Glaskeramiken mit einem oder mehreren weiteren Gläsern und/oder Glaskeramiken gemischt. Es ist auch die Mischung von zwei oder mehr erfindungsgemäßen Glaskeramiken möglich. Jede dieser Mischungen ist eine Mischglaskeramik, die mindestens eine erfindungsgemäße Glaskeramik enthält. Als weitere Komponenten zum Mischen mit der oder den erfindungsgemäßen Glaskeramiken eignen sich Gläser oder Glaskeramiken, je nachdem welche. Eigenschaften angestrebt werden. Die Herstellung der Mischglaskeramiken umfaßt die Schritte:
(A) Herstellen einer erfindungsgemäßen Glaskeramik, vorzugsweise gemäß den oben beschriebenen Schritten (a) bis (d),
(B) Herstellen eines weiteren Glaspulvers mit anderer Zusammensetzung oder eines weiteren Glaskeramikpulvers mit anderer Zusammensetzung und
(C) Mischen der Komponenten aus den Schritten (A) und (B).
(D) Wahlweise kann die so erhaltene Mischung thermisch nachbehandelt werden, um die Homogenität der Mischung zu gewährleisten.
(E) Die Pulvermischung kann zu kompakten Körpern gesintert oder alternativ zu monolithischen Körpern verpreßt und/oder gesintert werden.

Derartige Mischglaskeramiken stellen ein wichtiges Anwendungsgebiet für die erfindungsgemäßen Apatitglaskeramiken dar. Die Mischglaskeramiken zeichnen sich durch einen Gehalt an phosphat-und fluorfreien Kristallen vom Typ des silicatischen Oxyapatits aus. Ebenfalls Gegenstand der Erfindung sind mehrkomponentige Werkstoffe, die als erste Komponente mindestens eine erfindungsgemäße Apatitglaskeramik in Pulverform und als zweite Komponente mindestens ein weiteres Glas- und/oder Glaskeramikpulver enthalten. Diese Werkstoffe lassen sich auf die oben beschriebene Weise in Mischglaskeramiken überführen und erlauben es, durch einfaches Abmessen der Komponenten 1 und 2 eine Mischglaskeramik herzustellen, die beispielsweise einen bestimmten WAK aufweist.

Die erfindungsgemäßen phosphatfreien Apatitglaskeramiken sind mit praktisch allen Gläsern und Glaskeramiken frei mischbar, wodurch Mischglaskeramiken mit silicatischen Oxyapatitphasen entstehen. Es ist aber auch möglich, die erfindungsgemäße Apatitglaskeramik mit Silico-Phosphat-Gläsern in Pulver oder Granulatform zu mischen, ohne daß beim anschließenden Sintern der phosphatfreie, silicatische Oxyapatit in der erfindungsgemässen Glaskeramik zerstört wird.

Bei der oder den weiteren Glaskeramiken, die als Mischkomponente den erfindungsgemäßen Glaskeramiken zugesetzt werden, kann es sich um erfindungsgemäße oder nicht erfindungsgemäße Glaskeramiken handeln. Bevorzugte Gläser und nicht erfindungsgemäße Glaskeramiken zur Kombination mit den erfindungsgemäßen Glaskeramiken sind Silicat-, Borat-, Phosphat- oder Alumosilicatgläser und -glaskeramiken. Besonders bevorzugt sind SiO₂-Al₂O₃-K₂O- (mit kubischen oder tetragonalen Leucitkristallen), SiO₂-B₂O₃-Na₂O-, Alkalisilicat-, Alkali-Zink-Silicat-, Silicophosphat- und/oder SiO₂-ZrO₂-Gläser und -Glaskeramiken. Diese Mischungen enthalten vorzugsweise 5 bis 90 Gew.-% erfindungsgemäße Glaskeramik und 5 bis 90 Gew.-% nicht erfindungsgemäße Gläser und/oder Glaskeramiken. In den resultierenden Mischglaskeramiken liegt der Gehalt an Oxyapatitkristallen vorzugsweise im Bereich von 1 bis 35 Gew.-%.

Besonders geeignete Gläser und Glaskeramiken sowie deren Herstellung werden in DE 43 14 817, DE 44 23 793, DE 44 23 794, DE 196 47 739, DE 197 25 552, DE 197 25 553, DE 197 25 555, DE 100 31 431 und insbesondere DE 44 28 839 beschrieben, auf die hiermit ausdrücklich Bezug genommen wird.

Selbstverständlich können gesinterte oder monolithische Körper auch dadurch erhalten werden, daß ein oder mehrere erfindungsgemäße Glaskeramikpulver ohne den Zusatz nicht erfinderischer Gläser oder Glaskeramiken verwendet werden.

Ein als zweite Komponente besonders bevorzugtes Glas ist ein Alkali-Zink-Silikat-Glas, welche die folgenden Komponenten enthält:

| | |
|---|---|
| SiO₂ | 52,0 bis 63,5 Gew.-% |
| Me'[III]₂O₃ | 8,5 bis 13,0 Gew.-% |
| K₂O | 0 bis 20,5 Gew.-% |
| Na₂O | 1,5 bis 20,0 Gew.-% |
| Li₂O | 0 bis 5,0 Gew.-% |
| ZnO | 2,0 bis 8,0 Gew.-% |
| Me' [II]O | 2,5 bis 6,5 Gew.-% |
| TiO₂ + ZrO₂ | 0,5 bis 6,0 Gew.-% |
| SnO₂ | 0 bis 9,5 Gew.-% |
| P₂O₅ | 0 bis 4,0 Gew.-% |
| F | 0 bis 2,0 Gew.-% |
| CeO₂ | 0 bis 3,0 Gew.-%, |

wobei
(a) Me' [III]₂O₃ durch 0 bis 13 Gew.-% Al₂O₃ und 0 bis 9,5 Gew.-% La₂O₃.gebildet wird; und
(b) Me' [II]O durch 0 bis 3,5 Gew.-% CaO, 0 bis 4,5 Gew.-% BaO und 0 bis 5,0 Gew.-% MgO gebildet wird.

Die Herstellung diese Glases wird in der DE 44 28 839 beschrieben.

Zur Kombination mit den erfindungsgemäßen Glaskeramiken eignen sich ganz besonders Gläser und Glaskeramiken mit den nachstehend angegebenen Zusammensetzungen:
Bei niedriger Temperatur sinterndes Kalium-Zink-Silicat-Glas (DE 100 31 431) :

| Komponente | Anteil [in Gew.-%] |
|---|---|
| SiO₂ | 60,0 - 72,0 |
| Li₂O | 1,0 - 5,0 |
| K₂O | 10,0 - 23,0 |
| ZnO | 8,5 - 20,0 |

Bei niedriger Temperatur sinternde Apatitglaskeramik (DE 100 31 430):

| Komponente | Anteil [Gew.-%] |
|---|---|
| SiO₂ | 56,0 - 65,0 |
| Li₂O | 1,8 - 5,3 |
| K₂O | 9,0 - 17,5 |
| ZnO | 9,0 - 16,0 |
| CaO | 3,5 - 10,5 |
| P₂O₅ | 2,0 - 6,0 |
| F | 0,5 - 1,0 |

Transluzente Apatitglaskeramik (DE 197 25 555 / DE 197 25 553):

| Komponente | Anteil [Gew.-%] |
|---|---|
| SiO₂ | 45,0 - 70,0 |
| Al₂O₃ | 5, 0 - 22,0 |
| K₂O | 3,0 - 8,5 |
| Na₂O | 4,0 - 13,0 |
| CaO | 1,5 - 11,0 |
| P₂O₅ | 0,5 - 6,5 |
| F | 0,1 - 2,5 |

Alkalisilicatglas (DE 197 25 552):

| Komponente | Anteil [Gew.-%] |
|---|---|
| SiO₂ | 55,0 - 71,0 |
| Al₂O₃ | 5,0 - 16,0 |
| B₂O₃ | 0,2 - 10,0 |
| K₂O | 4,5 - 10,0 |
| Na₂O | 3,0 - 14,0 |

Sinterbare Lithiumdisilicatglaskeramik (DE 196 47 739):

| Komponente | Anteil [Gew.-%] |
|---|---|
| SiO₂ | 57,0 - 80,0 |
| Al₂O₃ | 3,0 - 5,0 |
| La₂O₃ | 0,1 - 6,0 |
| Li₂O | 11,0 - 19,0 |

ZrO₂ - SiO₂- Glaskeramik (DE 44 23 794) :

| Komponente | Anteil [Gew.-%] |
|---|---|
| SiO₂ | 42,5 - 58,5 |
| Li₂O | 7,0 - 14,5 |
| P₂O₅ | 4,0 - 13,5 |
| ZrO₂ | 15,0 - 28,0 |

Leucit enthaltende Phosphosilicatglaskeramik (DE 44 23 793):

| Komponente | Anteil [Gew.-%] |
|---|---|
| SiO₂ | 49,0 - 57,5 |
| Al₂O₃ | 11,4 - 21,0 |
| P₂O₅ | 0,5 - 5,5 |
| CaO | 2,5 - 11,5 |
| K₂O | 9,0 - 22,5 |
| Na₂O | 1,0 - 9,5 |
| ZrO₂ | 0,8 - 8,5 |
| F | 0,25 - 2,5 |

Opaleszierendes Glas (DE 43 14 817):

| Komponente | Anteil [Gew.-%] |
|---|---|
| SiO₂ | 48,0 - 66,0 |
| Me^{III}₂O₃ | 5,0 - 20,0 |
| Me^{I}₂O | 6,0 - 22,0 |
| Me^{II}O | 3,5 - 16,0 |
| Me^{IV}O₂ | 0,5 - 10,0 |
| P₂O₅ | 0,5 - 5,0 |

Die durch Mischen der erfindungsgemäßen Glaskeramiken mit den oben genannten Gläsern und Glaskeramiken erhaltenen Glaskeramiken sind ebenfalls Gegenstand der Erfindung.

Neben den genannten Komponenten können die erfindungsgemäßen Glaskeramiken und Mischglaskeramiken vorteilhaft ein oder mehrere farbgebende oder fluoreszenzgebende Oxide von Metallen enthalten, die vorzugsweise aus der Gruppe der 3d- und 4f-Elemente des Periodensystems der Elemente ausgewählt sind insbesondere ein oder mehrere Oxide der Metalle Zr, Ta, Yb, Nb, Tb, La, Er, Pr, Ce, Ti, V, Fe und Mn. Diese Oxide werden in die Glasstruktur eingebaut.

Die erfindungsgemäßen Apatitglaskeramiken eignen sich besonders als Dentalwerkstoffe und zur Herstellung von Dentalwerkstoffen, insbesondere Beschichtungsmaterialien oder Verblendmaterialien, und zur Herstellung von Dentalrestaurationen, insbesondere Kronen, Brücken, Teilkronen, Onlays, künstlichen Zähnen, Stumpfaufbauten oder Facetten. Darüber hinaus eignen sich die erfindungsgemäßen Apatitglaskeramiken insbesondere auch als Trübungskomponente für Dentalkomposite, Opaquer-Werkstoffe etc.

Im folgenden wird die Erfindung anhand von Beispielen und Figuren näher erläutert, wobei
Figur 1 eine elektronenmikroskopische Aufnahme einer erfindungsgemäßen Glaskeramik mit NaY₉(SiO₄)₆O₂-Kristallphase zeigt,
Figur 2 ein Röntgenbeugungsdiagramm der Glaskeramik aus Figur 1 zeigt,
Figur 3 eine elektronenmikroskopische Aufnahme einer weiteren erfindungsgemäßen Glaskeramik mit NaY₉(SiO₄)₆O₂-Kristallphase zeigt,
Figur 4 ein Röntgenbeugungsdiagramm der Glaskeramik aus Figur 3 zeigt,
Figur 5 eine mit einer erfindungsgemäßen Mischglaskeramik verblendete Dentalrestauration aus labialer Sicht und
Figur 6 dieselbe Dentalrestauration aus palatinaler Sicht zeigt.

### Ausführungsbeispiele

### Beispiele 1 bis 17: Herstellung von Apatitglaskeramiken

Es wurden insgesamt 17 verschiedene erfindungsgemäße Glaskeramiken hergestellt, indem eine homogene Mischung der zur Erzielung der in Tabelle I angegebenen Zusammensetzungen erforderlichen Rohstoffe hergestellt und bei Temperaturen von 1550°C zu einer Glasschmelze verarbeitet wurden. Die erhaltene Glasschmelze wurde in ein Wasserbad eingegossen und das dabei erhaltene Glasgranulat auf eine mittlere Korngröße von < 90 µm gemahlen. Das Glaspulver wurde dann einer zweistufigen thermischen Behandlung bei 800 °C (1 Stunde) und 1050 °C (30 Minuten) unterzogen.

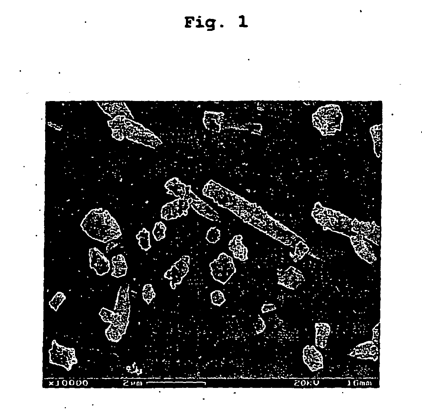

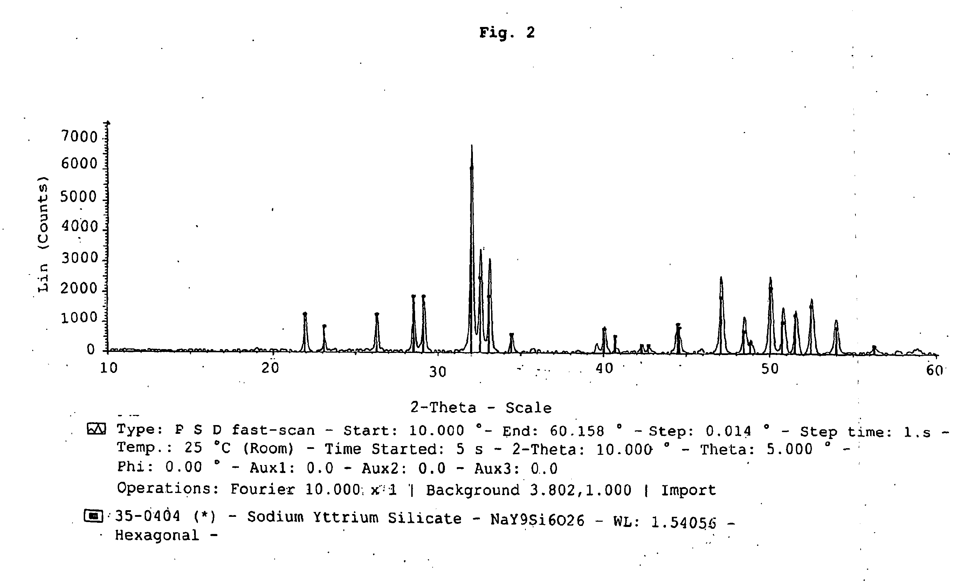

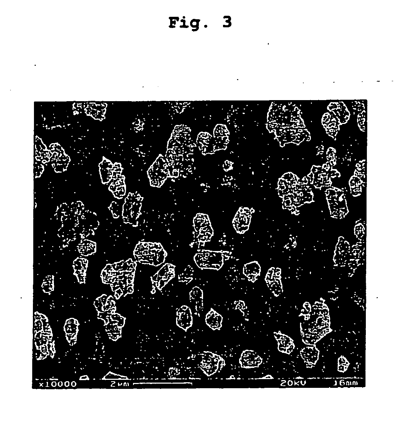

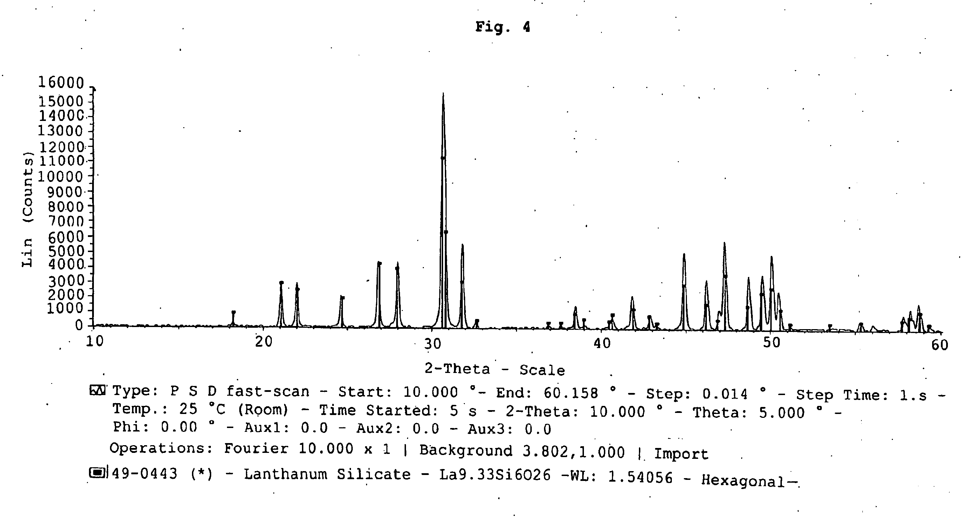

Anschließend wurden die Glaskeramiken durch Rasterelektronenmikroskopie und Röntgenbeugungsanalyse weiter untersucht. Die dabei gefundenen Kristallphasen sind ebenfalls in Tabelle I angegeben. Figur 1 zeigt eine elektronenmikroskopische Aufnahme der Glaskeramik aus Beispiel 2, Figur 3 eine Aufnahme der Glaskeramik aus Beispiel 12. In Figur 2 ist ein Röntgenbeugungsdiagramm der Glaskeramik aus Beispiel 2 gezeigt, in Figur 4 ein Röntgenbeugungsdiagramm der Glaskeramik aus Beispiel 12. Die Bestimmung der Kristallphasen erfolgte nach der JCPDS-Kartei, welche eine umfassende Datenbank für Röntgenbeugungsdiagramme kristalliner Verbindungen darstellt. Die jeweiligen Referenzen gemäß dieser Kartei sind als sogenannte Strichdiagramme in Fig.2 und Fig. 4 eingezeichnet. So enthält Fig. 2 das Referenzdiffraktogramm 35-0404 für NaY₉Si₆O₂₆ [NaY₉(SiO₄)₆O₂]. Die Übereinstimmung mit dem ermittelten Diffraktogramm der erfindungsgemäßen Apatitglaskeramik ist eindeutig.

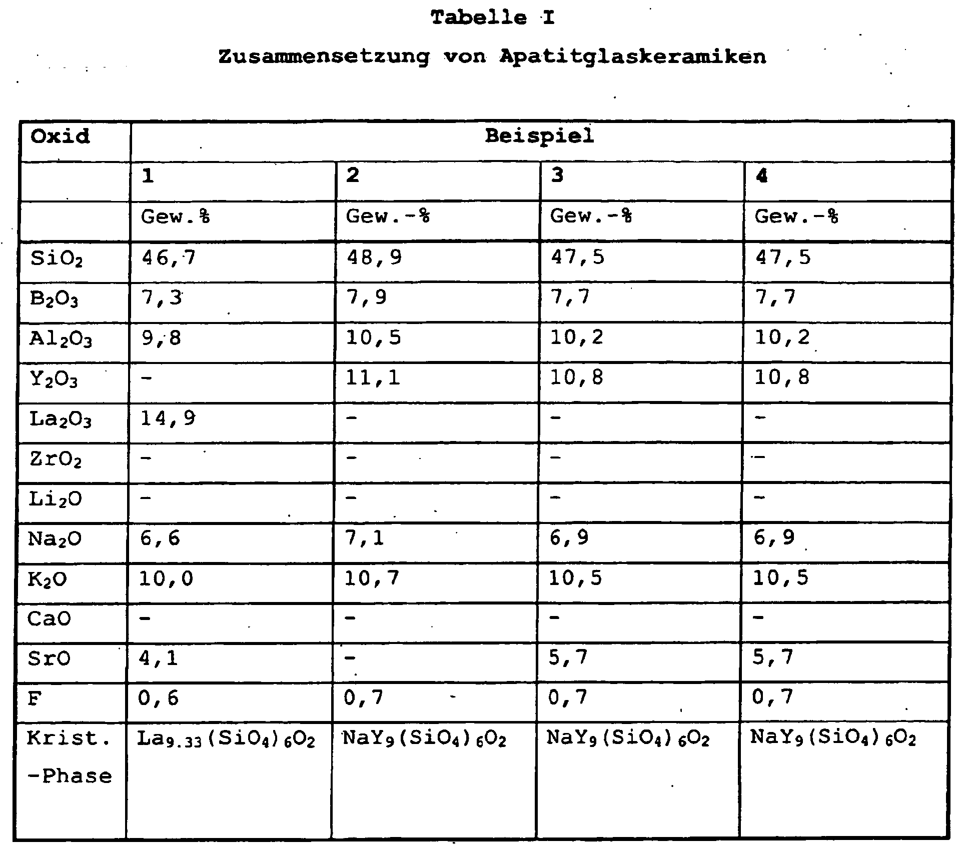

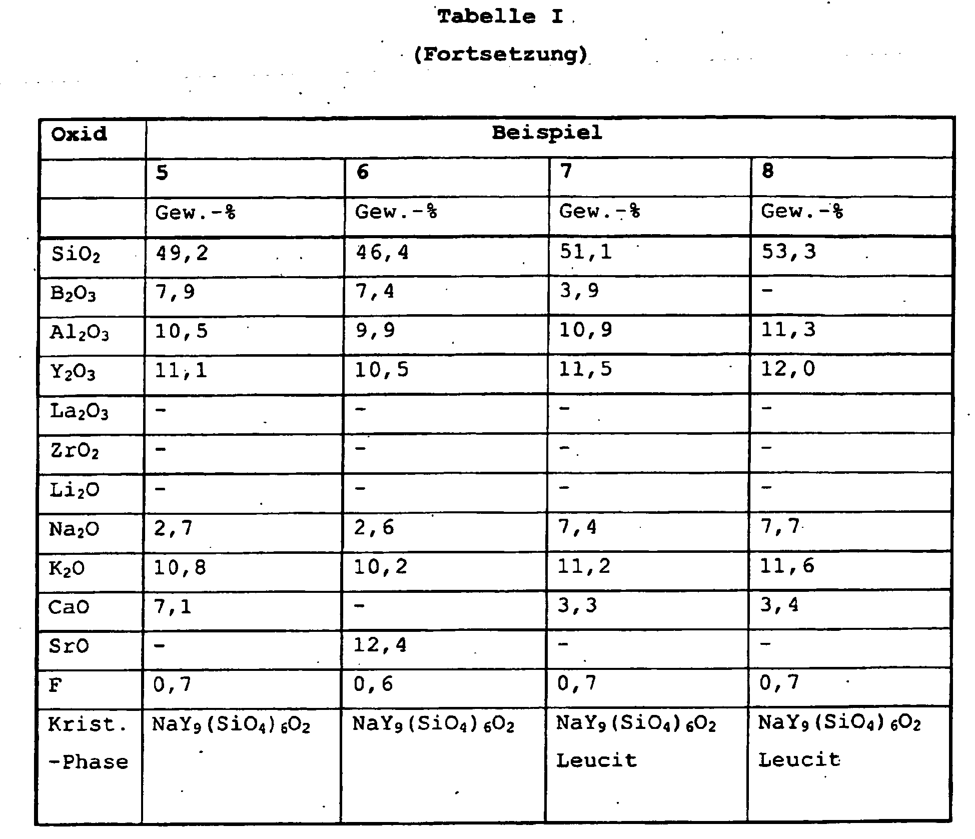

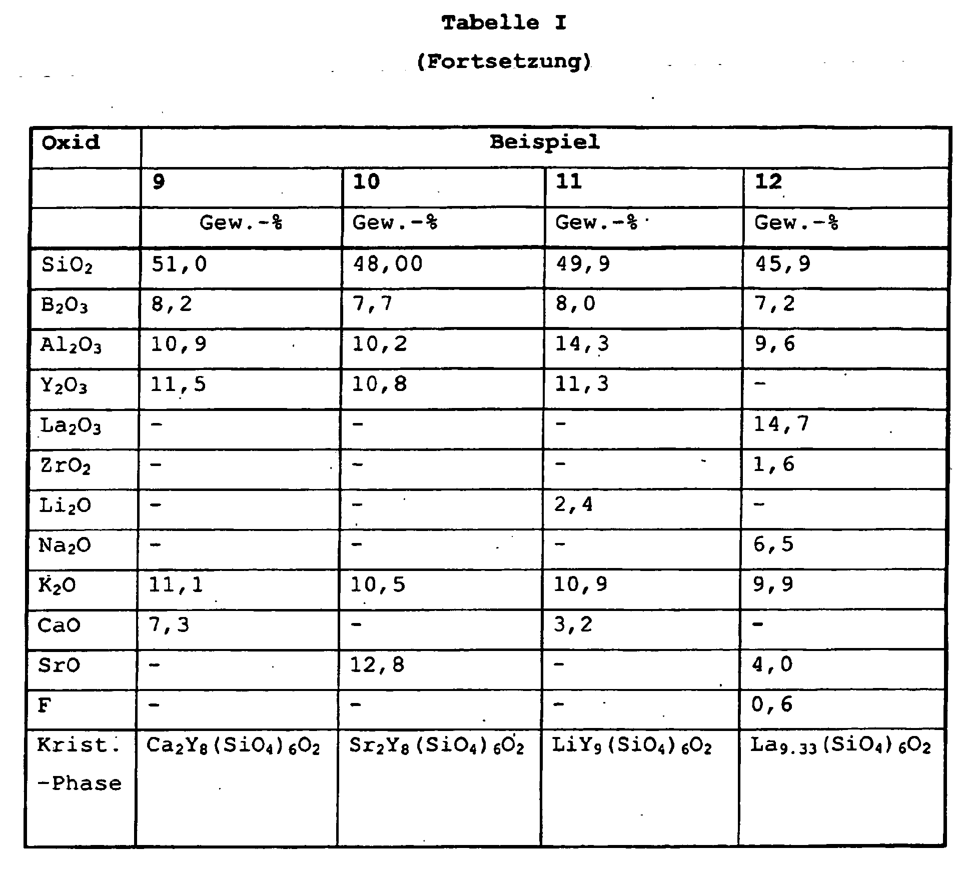

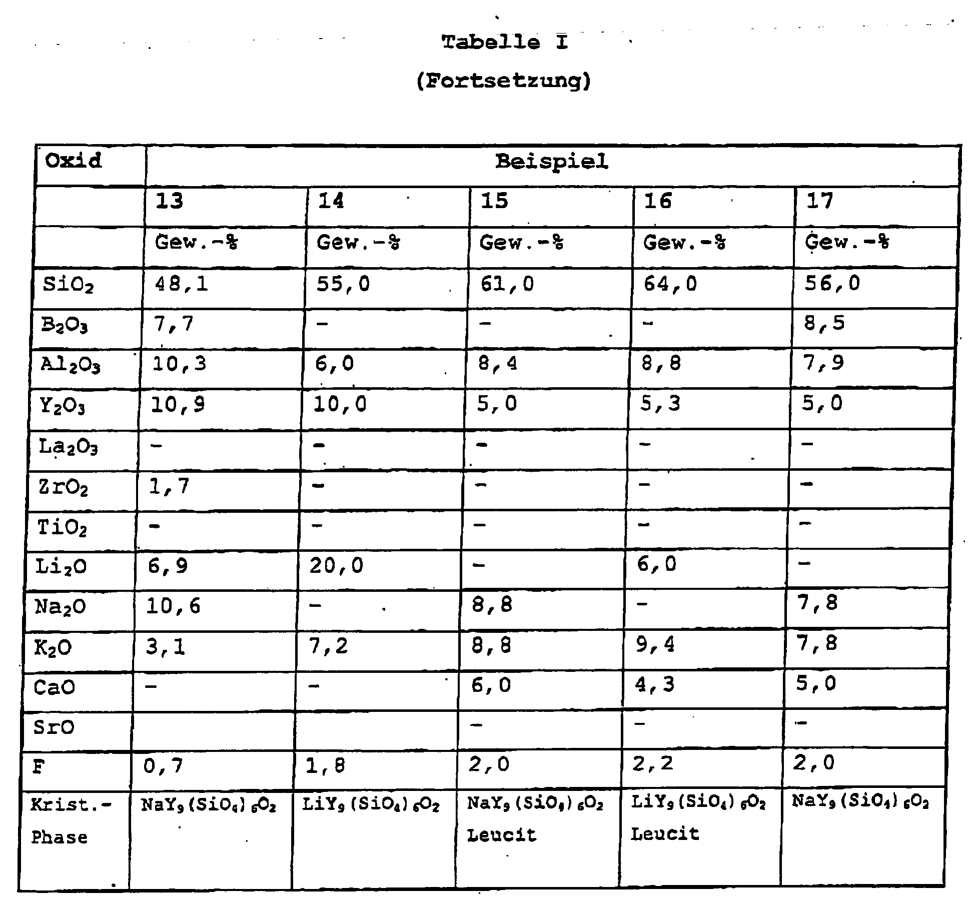

Die optischen und physikalischen Eigenschaften einiger Glaskeramiken sind in Tabelle II zusammengefaßt. Diese Eigenschaften wurden auf die in Beispiel 18 beschriebene Weise ermittelt. Der WAK der gezeigten Glaskeramiken reicht von 6,5 * 10⁻⁶ K⁻¹ (Beispiel 9) bis 14,1 * 10⁻⁶ K⁻¹ (Beispiel 6), die Brenntemperaturen von 830 °C (Beispiel 11) bis 1160 °C (Beispiel 9).

Die Glaskeramiken der Beispiele 1, 4, 8, 9 und 11 sind wegen ihrer niedrigeren WAK-Werte besonders für die Beschichtung und Verblendung von Al₂O₃-, ZrO₂- Keramiken, Titan oder Titanlegierungen und Lithiumdisilicat-Glaskeramiken mit einem niedrigen WAK-Bereich von 7 bis 12 * 10⁻⁶ K⁻¹ geeignet (siehe Tabelle II), die Glaskeramiken der Beispiele 7 und 8 eignen sich auf Grund ihrer hohem WAK-Werte besonders zur Beschichtung oder Verblendung von hochgoldhaltigen Dentallegierungen mit einem hohen WAK-Bereich von 14 bis 16* 10⁻⁶ K⁻¹. (siehe Tabelle II)

**Tabelle II**

| **Eingenschaften ausgewählter Glaskeramiken** | | | | | | | |
|---|---|---|---|---|---|---|---|
| **Parameter** | **Beispiel** | | | | | | |
| | **1** | **4** | **7** | **8** | **9** | **10** | **11** |
| Schmelzbedingungen | 1550°C für 2h | | | | | | |
| Optik nach Abschrecken | transparent | | | | | | |
| Temperbedingungen | 800°C für 1 h und 1050°C für 0,5 h | | | | | | |
| Optik nach Tempern | starke Trübung und hohe Helligkeit | | | | | | |
| Brenntemp. [°C] | 850 | -⁴ | 1010 | 1110 | 1010 | 1160 | 870 |
| WAK¹[10⁻⁶K⁻¹] | 9,7¹ | 9,5² | 14,1¹ | 13,3¹ | 7,1² | 6,5² | 9,4¹ |
| Tg²[°C] | 575 | 601 | 533 | 589 | 666 | 648 | 580 |
| Chem. Beständigkeit⁵ | -⁴ | -⁴ | 53,7 | 41,0 | -⁴ | -⁴ | 42,9 |

| | | | | | | | |
|---|---|---|---|---|---|---|---|
| ¹ Temperaturbereich : 100-500°C | | | | | | | |
| ² Temperaturbereich : 100-600°C | | | | | | | |
| ³ Transformationstemperatur | | | | | | | |
| ⁴ nicht ermittelt | | | | | | | |
| ⁵ Gemessen nach ISO 9693 | | | | | | | |

### Beispiel 18: Herstellung eines Verblendmaterials auf der Basis einer Mischglaskeramik

Es wurde eine Mischungsreihe aus mehreren zweikomponentigen Mischglaskeramiken erstellt. Die Zusammensetzungen der Mischglaskeramiken wurden so ausgewählt, daß durch Variation der Anteile der Mischungskomponenten eine systematische Eigenschaftsvariation resultierte. Ebenfalls miteinbezogen in die Untersuchung wurden die Randglieder der Mischungsreihe, also die reinen Komponenten A und B. Als Komponente A diente die Glaskeramik aus Beispiel 7, als Komponente B ein Glas der folgenden Zusammensetzung, das gemäß DE 44 28 839 hergestellt wurde (alle Angaben in Gew.-%):

**Tabelle III**

| **Zusammensetzung des Glases B** | |
|---|---|
| **Komponente** | **Anteil** |
| SiO₂ | 63,5 Gew.-% |
| Al₂O₃ | 8,8 Gew.-% |
| K₂O | 9, 6 Gew.-% |
| Na₂O | 6,8 Gew.-% |
| Li₂O | 0,8 Gew.-% |
| ZnO | 2,4 Gew.-% |
| CaO | 3,5 Gew.-% |
| TiO₂ | 0,4 Gew.-% |
| ZrO₂ | 1,0 Gew.-% |
| P₂O₅ | 0,4 Gew.-% |
| F | 0,8 Gew.-% |
| CeO₂ | 0,8 Gew.-% |
| B₂O₃ | 1,2 Gew.-% |

Es wurden die folgenden Mischungen hergestellt, thermisch behandelt und anschließend untersucht:

**Tabelle IV**

| **Zusammensetzung der untersuchten Glaskeramik-Mischungen** | | | |
|---|---|---|---|
| **Bezeichnung der Mischung** | **Gehalt an A [Gew.-%]** | **Gehalt an B [Gew.-%]** | **Temperatur der therm. Behand- lung [**°**C]** |
| M1 | 100,0 | 0,0 | 1020 |
| M2 | 80,0 | 20,0 | 900 |
| M3 | 50,0 | 50,0 | 840 |
| M4 | 20,0 | 80,0 | 770 |
| M5 | 0,0 | 100,0 | 740 |

Innerhalb der Reihe M1 bis M5 nimmt der Gehalt an der erfindungsgemässen Glaskeramik A ab während der Gehalt an Glas B zunimmt .

### Herstellung, Aufbereitung und Untersuchung der Mischungen:

Entsprechend der Tabelle IV wurden die einzelnen Komponenten eingewogen und innig miteinander vermischt. Anschließend wurden aus den Pulvern durch uniaxiales Pressen (max. 1 bar) zylindrische, konische Grünkörper mit einer Höhe von 35 mm und einem mittlerem Durchmesser von 40 mm hergestellt. Diese wurden über einen Zeitraum von 1 Stunde bei den in Tabelle IV angegebenen Temperaturen thermisch behandelt. Nach Ablauf dieser Zeitspanne wurden die gesinterten Körper in kaltem Wasser abgeschreckt, getrocknet, mechanisch zerkleinert und auf ein Korngröße < 90µm abgesiebt.

Die erfindungsgemässen Glaskeramiken und die daraus hergestellten Mischglaskeramiken wurden den folgenden Tests unterworfen:

### Bestimmung des linearen thermischen Äusdehnungskoeffizienten (WAK):

Zur Messung des linearen thermischen Ausdehnungskoeffizienten (WAK) wurde aus den Pulvern der jeweiligen Glaskeramiken stäbchenförmige Grünkörper hergestellt, die in einem Vakuumbrennofen (Programat® P100, Ivoclar Vivadent AG) mit einer Aufreizrate von 60°C/Min. und einer Haltezeit von 1 Minute bei den in den Tabellen II und VI angegebenen Brenntemperaturen gesintert wurden. Anschließend wurde ein Glanzbrand ohne Vakuum bei einer um 20°C höheren Endtemperatur und einer Haltezeit von 1 Minute durchgeführt. An dem erhaltenen Probekörper wurde der lineare thermische Ausdehnungskoeffizient im Temperaturbereich von 100 bis 500°C oder 600°C mit einem Dilatometer (Firma Bähr, Typ 803) bestimmt. Die Ergebnisse sind in den Tabellen II und VI gezeigt.

### Bestimmung der chemischen Beständigkeit:

Die chemische Beständigkeit wurde gemäß der ISO-Vorschrift 9693 bestimmt. Dazu wurden zunächst Probeplättchen mit einem Durchmesser von 12 mm und einer Dicke von 1 mm durch Zusammensintern von Glaskeramik-Pulver mit einer Teilchengrösse von < 90 µm im Vakuumbrennofen (Programat® P100, Ivoclar Vivadent AG) hergestellt. Die Probekörper wurden 1 Minute lang bei den in den Tabellen II und VI angegebenen Brenntemperatur gesintert. Dann wurden die Plättchen 16 Stunden in 4 Vol.-%iger wäßriger Essigsäure in geschlossenen Glasgefäßen bei 80°C gelagert. Als Maß für die chemische Beständigkeit wurde der gemessene Masseverlust bezogen auf die Plättchenoberfläche bestimmt. Die Ergebnisse sind in den Tabellen II und VI gezeigt.

### Bestimmung der Brenntemperatur:

Als Brenntemperatur wurde die Temperatur angenommen, bei der ein Standardplättchen durchgesintert ist und einen leichten Oberflächenglanz aufweist. Das Plättchen wird durch manuelles Verdichten des mit Wasser angefeuchteten Pulvers in einer Metallform (Ø = 16 mm, h = 1,6 mm) und anschließendem einmaligen Brennen hergestellt. Die Ergebnisse sind in den Tabellen II und VI gezeigt.

### Bestimmung der Biegefestigkeit:

Die Biegefestigkeit wurde auf die in der Norm ISO 6872 beschriebene Weise in einem biaxialen Versuchsaufbau ermittelt. Zu diesem Zweck wurden 8 Standardplättchen aus angefeuchteter, manuell verdichteter Keramik durch zweifaches Brennen hergestellt, die nach planparalleler Bearbeitung auf eine Dicke von 1,2 mm poliert wurden. Die Ergebnisse sind in Tabelle VI gezeigt.

### Bestimmung der Transluzenz:

Die Bestimmung der Transluzenz (ΔCR-Wert) wurde gemäß der Norm BS 5612 durchgeführt. Durch hydraulisches Verpressen von 2,5 g Pulver in einem genormten Werkzeug wurden Grünkörper hergestellt, die nach zweifachem Brand planparallel bearbeitet und poliert werden. Die Ergebnisse sind in Tabelle VI gezeigt.

**Tabelle VI**

| **Eigenschaften der Glasskeramiken nach dem Aufbrennen auf Dantellegierung** | | | | | | |
|---|---|---|---|---|---|---|
| **Nr.** | **Brenn- temp. [°C]** | **WAK¹ [10⁻⁶·K⁻¹]** | **T_{g}² [°C]** | **Chem. Best.³ [µg cm⁻²]** | **Biegef.⁴ [MPa]** | **Transl.⁵ ΔCR** |
| M1 | 990 | 14,1 | 533 | 53,7 | 116 | 0,98 |
| M2 | 960 | 12,9 | 558 | 16,5 | 112 | 0,97 |
| M3 | 890 | 13,1 | 532 | 24,6 | 109 | 0,96 |
| M4 | 850 | 11,1 | 516 | 23,9 | 78 | 0,88 |
| M5 | 840 | 9,2 | 510 | 21,0 | 93 | 0,27 |

| | | | | | | |
|---|---|---|---|---|---|---|
| ¹ linearer thermischer Ausdehnungskoeffizient im Temperaturbereich von 100 bis 500 °C | | | | | | |
| ² Transformationstemperatur | | | | | | |
| ³ chemische Beständigkeit gemäß EN ISO 9693 | | | | | | |
| ⁴ Biegefestigkeit gemäß ISO 6872 | | | | | | |
| ⁵ Transluzenz bestimmt nach BS 5612-1978 | | | | | | |

Die in Tabelle VI gezeigten Eigenschaften verhalten sich innerhalb der Mischungsreihe nahezu linear in Abhängigkeit von der Zunahme bzw. Abnahme einer Komponente und können daher durch Wahl der Zusammensetzung einer Mischung eingestellt werden.

Die Ergebnisse zeigen, daß Brenntemperatur, linearer thermischer Ausdehnungskoeffizient (WAK), chemische Beständigkeit, Biegefestigkeit und Transluzenz in einfacher Weise durch eine Mischungsreihe eingestellt werden können, die aus unterschiedlichen Mischungen beispielsweise von zwei Ausgangskomponenten A und B gebildet werden kann. Jede dieser Mischungen stellt eine Mischglaskeramik dar.

### Bestimmung der Benetzung:

Die Benetzung des Gerüstmaterials durch die Mischungen wurde an Mischungen überprüft, die aufgrund der Brenntemperatur und des thermischen Ausdehnungskoeffizienten für ein gegebenes Gerüstmaterial geeignet erschienen. Zu diesem Zweck wurden standardisierte dreigliedrige Frontzahnbrücken aus einer Palladiumbasislegierung als Gerüst gewählt, die folgende Werkstoffcharakteristiken aufwiesen:

**Tabelle V**

| **Eigenschaften und Zusammensetzung der Dentallegierung** | | | |
|---|---|---|---|
| **Chemische Zusammen- setzung [Gew. %]** | **WAK_{(25-500 °C)} [10⁻⁶ K⁻¹]** | **Dichte [g cm⁻³]** | **Soliduspunkt [°C]** |
| Pd 75,2 | 13,8 | 11,0 | 1270 |
| Au 6,0 | | | |
| Ag 6,5 | | | |
| In 6,0 | | | |
| Ga 6,0 | | | |
| Ru < 1,0 | | | |
| Li < 1,0 | | | |

Entsprechend der zahntechnischen Arbeitsweise wurden diese Brükken konditioniert und anschließend durch Grundieren mit einem Opaquer zur Verblendung mit der entsprechenden Mischung vorbereitet. Anschließend wurde die aufzutragende Glaskeramikmischung mit Wasser geschlickert und auf das Metallgerüst aufgetragen. Das Sintern erfolgte in einem Dentalbrennofen unter Vakuum nach den in Tabelle VI angegebenen Brennparametern. Der Opaquer dient zur Grundierung und Abdeckung der Eigenfarbe des Metalls. Als Opaquer wurden nicht erfinderische Glaskeramiken, die mit feinst verteilten Farbpigmenten versetzt waren, in dünner Schicht bei 840 °C auf das Metallsubstrat aufgesintert.

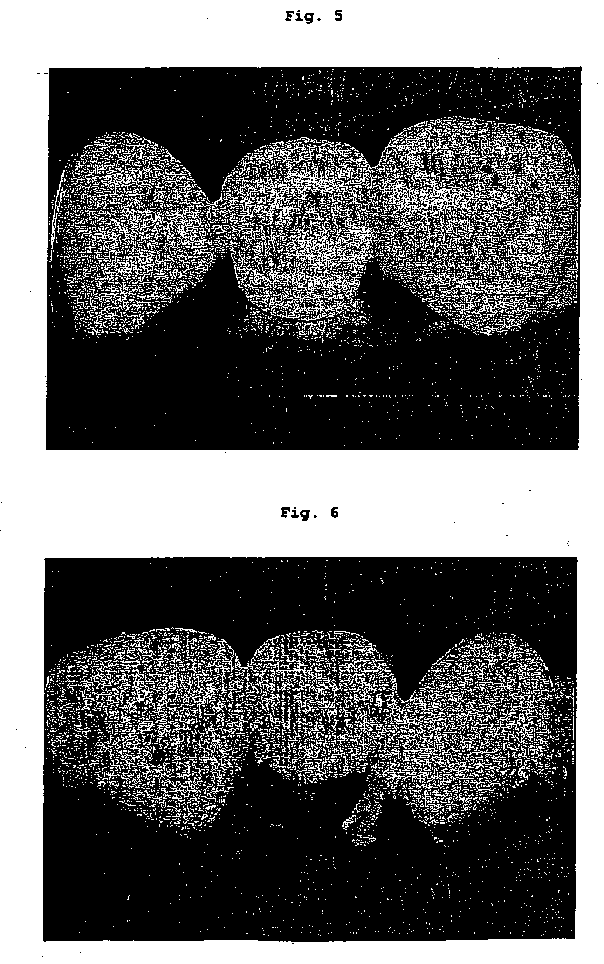

Um die Benetzungsgrad beurteilen zu können, wurde nur ein Sintervorgang, der sogenannte Hauptbrand, durchgeführt. Die Bewertung erfolgte qualitativ und wurde fotografisch dokumentiert. Das Benetzungsverhalten der Mischglaskeramik ist am Beispiel der Zusammensetzung M4 in Figur 5 und Figur 6 dargestellt. In Figur 5 ist eine mit der Mischglaskeramik M4 verblendete, dentale Brücke aus labialer Sicht gezeigt, in Figur 6 ist dieselbe Restauration aus palatinaler Sicht zu sehen. Es ist deutlich zu erkennen, daß sich das keramische Schichtmaterial innig mit der Opaquerschicht verbindet und diese vollständig benetzt. Weder an den dünn auslaufenden Rändern der Restauration noch im Interdentalbereich kommt es zu einer Abhebung des Schichtmaterials.

## Patentansprüche

1. Apatitglaskeramik mit mindestens einer Glasphase und mindestens einer Apatitphase, **dadurch gekennzeichnet, daß**
mindestens eine der Apatitphasen eine phosphat- und fluorfreie silicatische Oxyapatitphase der Formel Me[I]ₓMe[II]_{y}Me[III]_{z}(SiO₄)₆O₂ ist, in der Me[I] Na oder Li, Me[II] Ca oder Sr und Me[III] Y oder La ist und x, y und z so gewählt sind, daß die Summe der Wertigkeiten der Kationen Me[I], Me[II] und Me[III] den Wert 28 ergibt, und
die Apatitglaskeramik die folgenden Komponenten enthält, wobei sich die Prozentangaben auf die Gesamtmasse der Glaskeramik beziehen:
| | |
|---|---|
| SiO₂ | 40 bis 70 Gew.-% |
| B₂O₃ | 0 bis 9 Gew.-% |
| Al₂O₃ | 7 bis 15 Gew.-% |
| Y₂O₃ | 0 bis 14 Gew.-% |
| La₂O₃ | 0 bis 17 Gew.-% |
| ZrO₂ | 0 bis 3 Gew.-% |
| Li₂O | 0 bis 8 Gew.-% |
| Na₂O | 0 bis 24 Gew.-% |
| K₂O | 0 bis 14 Gew.-% |
| CaO | 0 bis 9 Gew.-% |
| SrO | 0 bis 15 Gew.-% |
| F | 0 bis 3 Gew.-%. |

2. Apatitglaskeramik nach Anspruch 1, in der alle Apatitphasen phosphat- und fluorfreie silicatische Oxyapatite sind.

3. Apatitglaskeramik nach Anspruch 1 oder 2, in der die eine oder mehreren Oxyapatitphasen die Formel Me[I]Me[III]₉(SiO₄)₆O₂, Me[II]₂Me[III]₈(SiO₄)₆O₂ und/oder Me[III]_{9,33}(SiO₄)₆O₂ haben.

4. Apatitglaskeramik nach Anspruch 3, in der die eine oder mehreren Oxyapatitphasen die Formel NaY₉(SiO₄)₆O₂, LiY₉(SiO₄)₆O₂, Sr₂Y₈(SiO₄)₆O₂, Ca₂Y₈(SiO₄)₆O₂, Sr₂La₈(SiO₄)₆O₂ und/oder La_{9,33}(SiO₄)₆O₂ haben.

5. Apatitglaskeramik nach.einem der Ansprüche 1 bis 4, die mindestens eine weitere kristalline Phase enthält.

6. Apatitglaskeramik nach Anspruch 5, die als weitere kristalline Phase Leucit enthält.

7. Apatitglaskeramik nach einem der Ansprüche 1 bis 6, welche die folgenden Komponenten enthält:
| | |
|---|---|
| SiO₂ | 45,9 bis 64,0 Gew.-% |
| B₂O₃ | 0 bis 8,5 Gew.-% |
| Al₂O₃ | 7,9 bis 14,3 Gew.-% |
| Y₂O₃ | 0 bis 12,0 Gew.-% |
| La₂O₃ | 0 bis 14,9 Gew.-% |
| ZrO₂ | 0 bis 1,7 Gew.-% |
| Li₂O | 0 bis 6, 0 Gew.-% |
| Na₂O | 0 bis 20,0 Gew.-% |
| K₂O | 0 bis 11,6 Gew.-% |
| CaO | 0 bis 7,3 Gew.-% |
| SrO | 0 bis 12,8 Gew.-% |
| F | 0 bis 2,2 Gew.-%. |

8. Apatitglaskeramik nach Anspruch einem der Ansprüche 1 bis 7, die ein oder mehrere farbgebende oder fluoreszenzgebende Metalloxide enthält.

9. Apatitglaskeramik nach Anspruch 8, in der das Metall des Metalloxids oder die Metalle der Metalloxide aus der Gruppe der 3d- und 4f-Elemente des Periodensystems der Elemente ausgewählt sind.

10. Apatitglaskeramik nach einem der Ansprüche 1 bis 9, in welcher der Anteil der Apatitkristalle mindestens 5 Gew.-% beträgt.

11. Apatitglaskeramik nach einem der Ansprüche 1 bis 10, in welcher die Apatitkristalle.nadelförmig sind.

12. Apatitglaskeramik nach einem der Ansprüche 1 bis 11, in welcher die Apatitkristalle eine durchschnittliche Länge von 0,1 bis 10 µm aufweisen.

13. Apatitglaskeramik nach einem der Ansprüche 1 bis 12, die im Temperaturbereich von 100 bis 500 °C einen Wärmeausdehnungskoeffizienten von 6 * 10⁻⁶ K⁻¹ bis 15 * 10⁻⁶ K⁻¹ aufweist.

14. Apatitglaskeramik nach einem der Ansprüche 1 bis 13, die eine Brenntemperatur von 830 °C bis 1150 °C aufweist.

15. Apatitglaskeramik nach einem der Ansprüche 1 bis 14, die eine chemische Beständigkeit von < 80 µg/cm² aufweist.

16. Verfahren zur Herstellung einer Apatitglaskeramik gemäß einem der Ansprüche 1 bis 15, bei dem man
(a) die zur Herstellung der Glaskeramik erforderlichen Komponenten mischt und zu einem Glas aufschmilzt,
(b) man die Glasschmelze aus Schritt (a) in ein Glasgranulat überführt,
(c) man ggf. das Glasgranulat zu einem Pulver mit einer mittleren Korngröße von 1 bis 500 µm (bezogen auf die Teilchenzahl) zerkleinert, und
(d) man das Glasgranulat aus Schritt (b) oder das Glaspulver aus Schritt (c) anschließend einer ein- oder mehrstufigen thermischen Behandlung im Temperaturbereich von 700 bis 1200 °C für eine Dauer von 30 Minuten bis 6 Stunden unterwirft.

17. Verfahren nach Anspruch 16, bei man das Glasgranulat aus Schritt (b) oder das Glaspulver aus Schritt (c) in Schritt (d) einer thermischen Behandlung im Temperaturbereich von 900 bis 1200 °C unterwirft.

18. Verfahren nach Anspruch 16 oder 17, bei man das Glasgranulat aus Schritt (b) oder das Glaspulver aus Schritt (c) in Schritt (d) einer thermischen Behandlung für eine Dauer von 30 Minuten bis 3 Stunden unterwirft.

19. Mehrkomponentiger Werkstoff, der als erste Komponente mindestens eine Apatitglaskeramik gemäß einem der Ansprüche 1 bis 15 in Pulverform und als zweite Komponente mindestens ein weiteres Glas- und/oder Glaskeramikpulver enthält.

20. Werkstoff nach Anspruch 19, der als zweite Komponente ein Alkali-Zink-Silikat-Glas enthält, welche die folgenden Komponenten enthält:
| | |
|---|---|
| SiO₂ | 52,0 bis 63,5 Gew.-% |
| Me'[III]₂O₃ | 8,5 bis 13,0 Gew.-% |
| K₂O | 0 bis 20,5 Gew.-% |
| Na₂O | 1,5 bis 20,0 Gew.-% |
| Li₂O | 0 bis 5,0 Gew.-% |
| ZnO | 3,6 bis 8,0 Gew.-% |
| Me'[II]O | 2,5 bis 6,5 Gew.-% |
| TiO₂ + ZrO₂ | 0,5 bis 6,0 Gew.-% |
| SnO₂ | 0 bis 9,5 Gew.-% |
| P₂O₅ | 0 bis 4, 0 Gew.-% |
| F | 0 bis 2,0 Gew.-% |
| CeO₂ | 0 bis 3,0 Gew.-%, |
wobei
(a) Me'[III]₂O₃ durch 0 bis 13.Gew.-% Al₂O₃ und 0 bis 9,5 Gew.-% La₂O₃.gebildet wird; und
(b) Me'[II]O durch 0 bis 3,5 Gew.-% CaO, 0 bis 4,5 Gew.-% BäO und 0 bis 5,0 Gew.-% MgO gebildet wird.

21. Mischglaskeramik, dadurch erhältlich, daß man die Komponenten eines Werkstoffs gemäß einem der Ansprüche 19 bis 20 mischt und dann in eine Mischglaskeramik überführt.

22. Verwendung einer Apatitglaskeramik gemäß einem der Ansprüche 1 bis 15 oder einer Mischglaskeramik gemäß Anspruch 21 als Trübungskomponente, Dentalwerkstoff, zur Herstellung eines Dentalwerkstoffs oder einer Dentalrestauration.

23. Verwendung nach Anspruch 22, wobei der Dentalwerkstoff ein Beschichtungsmaterial oder Verblendmaterial ist.

24. Verwendung nach Anspruch 22, wobei die Dentalrestauration eine Krone, Brücke, Teilkrone, ein Onlay, ein künstlicher Zahn, ein Stumpfaufbau oder eine Facette ist.

## Claims

1. Apatite glass ceramic comprising at least one glass phase and at least one apatite phase, wherein at least one of the apatite phases is a phosphate-free and fluorine-free siliceous oxyapatite phase of the formula Me[I]ₓMe[II]_{y}Me[III]_{z}(SiO₄)₆O₂, in which Me[I] is Na or Li, Me[II] is Ca or Sr and Me[III] is Y or La and x, y and z are chosen such that the sum of the valences of the cations Me[I], Me[II] and Me[III] yields the value 28, and
the apatite glass ceramic comprises the following components, wherein the percentages refer to the total weight of the glass ceramic:
| | |
|---|---|
| SiO₂ | 40 to 70 wt.-% |
| B₂O₃ | 0 to 9 wt.-% |
| Al₂O₃ | 7 to 15 wt.-% |
| Y₂O₃ | 0 to 14 wt.-% |
| La₂O₃ | 0 to 17 wt.-% |
| ZrO₂ | 0 to 3 wt.-% |
| Li₂O | 0 to 8 wt.-% |
| Na₂O | 0 to 24 wt.-% |
| K₂O | 0 to 14 wt.-% |
| CaO | 0 to 9 wt.-% |
| SrO | 0 to 15 wt.-% |
| F | 0 to 3 wt.-%. |

2. Apatite glass ceramic according to claim 1, wherein all the apatite phases are phosphate-free and fluorine-free siliceous oxyapatites.

3. Apatite glass ceramic according to claim 1 or 2, in which the one or plurality of oxyapatite phases have the formula Me[I]Me[III]₉(SiO₄)₆O₂, Me[II]₂Me[III]₈(SiO₄)₆O₂ and/or Me[III]_{9.33}(SiO₄)₆O₂.

4. Apatite glass ceramic according to claim 3, in which the one or plurality of oxyapatite phases have the formula NaY₉(SiO₄)₆O₂, LiY₉(SiO₄)₆O₂, Sr₂Y₈(SiO₄)₆O₂, Ca₂Y₈(SiO₄)₆O₂, Sr₂La₈(SiO₄)₆O₂ and/or La_{9.33}(SiO₄)₆O₂.

5. Apatite glass ceramic according to one of claims 1 to 4, comprising at least one further crystalline phase.

6. Apatite glass ceramic according to claim 5, which comprises leucite as a further crystalline phase.

7. Apatite glass ceramic according to one of claims 1 to 6, which comprises the following components:
| | |
|---|---|
| SiO₂ | 45.9 to 64.0 wt.-% |
| B₂O₃ | 0 to 8.5 wt.-% |
| Al₂O₃ | 7.9 to 14.3 wt.-% |
| Y₂O₃ | 0 to 12.0 wt.-% |
| La₂O₃ | 0 to 14.9 wt.-% |
| ZrO₂ | 0 to 1.7 wt.-% |
| Li₂O | 0 to 6.0 wt.-% |
| Na₂O | 0 to 20.0 wt.-% |
| K₂O | 0 to 11.6 wt.-% |
| CaO | 0 to 7.3 wt.-% |
| SrO | 0 to 12.8 wt.-% |
| F | 0 to 2.2 wt.-%. |

8. Apatite glass ceramic according to one of claims 1 to 7, which comprises one or a plurality of chromophoric or fluorophoric metal oxides.

9. Apatite glass ceramic according to claim 8 in which the metal of the metal oxide or the metals of the metal oxides are selected from the group of the 3d and 4f elements of the periodic table of the elements.

10. Apatite glass ceramic according to one of claims 1 to 9, in which the proportion of apatite crystals is at least 5 wt.-%.

11. Apatite glass ceramic according to one of claims 1 to 10, in which the apatite crystals are needle-shaped.

12. Apatite glass ceramic according to one of claims 1 to 11, in which the apatite crystals have an average length of 0.1 to 10 µm.

13. Apatite glass ceramic according to one of claims 1 to 12, which has a coefficient of thermal expansion of 6*10⁻⁶ K⁻¹ to 15*10⁻⁶ K⁻¹ in the temperature range of 100 to 500 °C.

14. Apatite glass ceramic according to one of claims 1 to 13, which has a firing temperature of 830 °C to 1150 °C.

15. Apatite glass ceramic according to one of claims 1 to 14, which has a chemical resistance of < 80 µg/cm².

16. Method for the production of an apatite glass ceramic according to one of claims 1 to 15, in which
(a) the components required for the production of the glass ceramic are mixed and are melted to form a glass,
(b) the glass melt from step (a) is transformed into a glass granulate,
(c) the glass granulate is optionally ground to a powder with an average grain size of 1 to 500 µm, (relative to the particle count), and
(d) the glass granulate from step (b) or the glass powder from step (c) is then subjected to a single-stage or multi-stage thermal treatment in the temperature range 700 to 1200 °C for a period of 30 minutes to 6 hours.

17. Method according to claim 16 in which the glass granulate from step (b) or the glass powder from step (c) is subjected to a thermal treatment in the temperature range of 900 to 1200 °C in step (d).

18. Method according to claim 16 or 17, in which the glass granulate from step (b) or the glass powder from step (c) is subjected to a thermal treatment for a period of 30 minutes to 3 hours in step (d).

19. Multi-component material comprising, as the first component, at least one apatite glass ceramic according to one of the claims 1 to 15 in powder form and, as the second component, comprises at least one further glass and/or glass ceramic powder.

20. Material according to claim 19, which comprises, as the second component, an alkali-zinc-silicate glass, which comprises the following components:
| | |
|---|---|
| SiO₂ | 52.0 to 63.5 wt.-% |
| Me'[III]₂O₃ | 8.5 to 13.0 wt.-% |
| K₂O | 0 to 20.5 wt.-% |
| Na₂O | 1.5 to 20.0 wt.-% |
| Li₂O | 0 to 5.0 wt.-% |
| ZnO | 3.6 to 8.0 wt.-% |
| Me'[II]O | 2.5 to 6.5 wt.-% |
| TiO₂ + ZrO₂ | 0.5 to 6.0 wt.-% |
| SnO₂ | 0 to 9.5 wt.-% |
| P₂O₅ | 0 to 4.0 wt.-% |
| F | 0 to 2.0 wt.-% |
| CeO₂ | 0 to 3.0 wt.-%, |
wherein
(a) Me'[III]₂O₃ is formed from 0 to 13 wt.-% Al₂O₃ and 0 to 9.5 wt.-% La₂O₃ and
(b) Me'[II]O is formed from 0 to 3.5 wt.-% CaO, 0 to 4.5 wt.-% BaO and 0 to 5.0 wt.-% MgO.

21. Mixed glass ceramic, obtained by mixing the components of a material according to one of claims 19 to 20 and then transforming them into a mixed glass ceramic.

22. Use of an apatite glass ceramic according to one of claims 1 to 15 or a mixed glass ceramic according to claim 21 as an opacifying component, a dental material, for the production of a dental material or a dental restoration.

23. Use according to claim 22, wherein the dental material is a coating material or facing material.

24. Use according to claim 22, wherein the dental restoration is a crown, bridge, partial crown, an onlay, an artificial tooth, a stump superstructure or a facet.

## Revendications

1. Vitrocéramique à apatite, comportant au moins une phase de verre et au moins une phase d'apatite, **caractérisée en ce qu'**au moins l'une des phases d'apatite est une phase d'oxyapatite silicatée exempte de phosphate et de fluor, de formule Me[I]ₓMe[II]_{y}Me[III]_{z}(SiO₄)₆O₂, dans laquelle Me[I] est Na ou Li, Me[II] est Ca ou Sr et Me[III] est Y ou La et x, y et z sont choisis de manière que la somme des valences des cations Me[I], Me[II] et Me[III] donne la valeur 28, et
la vitrocéramique à apatite contient les composants suivants, les pourcentages se rapportant à la masse totale de la vitrocéramique :
| | |
|---|---|
| SiO₂ | 40 à 70 % en poids |
| B₂O₃ | 0 à 9 % en poids |
| Al₂O₃ | 7 à 15 % en poids |
| Y₂O₃ | 0 à 14 % en poids |
| La₂O₃ | 0 à 17 % en poids |
| ZrO₂ | 0 à 3 % en poids |
| Li₂O | 0 à 8 % en poids |
| Na₂O | 0 à 24 % en poids |
| K₂O | 0 à 14 % en poids |
| CaO | 0 à 9 % en poids |
| SrO | 0 à 15 % en poids |
| F | 0 à 3 % en poids. |

2. Vitrocéramique à apatite selon la revendication 1, dans laquelle toutes les phases d'apatite sont des oxyapatites silicatées exemptes de phosphate et de fluor.

3. Vitrocéramique à apatite selon la revendication 1 ou 2, dans laquelle la ou les phase(s) d'oxyapatite ont la formule Me[I]Me[III]₉(SiO₄)₆O₂, Me[II]₂Me[III]₈(SiO₄)₆O₂ et/ou Me[III]_{9,33}(SiO₄)₆O₂.

4. Vitrocéramique à apatite selon la revendication 3, dans laquelle la ou les phase(s) phases d'oxyapatite ont la formule NaY₉(SiO₄)₆O₂, LiY₉(SiO₄)₆O₂, Sr₂Y₈(SiO₄)₆O₂, Ca₂Y₈(SiO₄)₆O₂, Sr₂La₈(SiO₄)₆O₂ et/ou La_{9,33}(SiO₄)₆O₂.

5. Vitrocéramique à apatite selon l'une quelconque des revendications 1 à 4, qui contient au moins une phase cristalline supplémentaire.

6. Vitrocéramique à apatite selon la revendication 5, qui contient de la leucite à titre de phase cristalline supplémentaire.

7. Vitrocéramique à apatite selon l'une quelconque des revendications 1 à 6, qui contient les composants suivants :
| | |
|---|---|
| SiO₂ | 45,9 à 64,0 % en poids |
| B₂O₃ | 0 à 8,5 % en poids |
| Al₂O₃ | 7,9 à 14,3 % en poids |
| Y₂O₃ | 0 à 12,0 % en poids |
| La₂O₃ | 0 à 14,9 % en poids |
| ZrO₂ | 0 à 1,7 % en poids |
| Li₂O | 0 à 6,0 % en poids |
| Na₂O | 0 à 20,0 % en poids |
| K₂O | 0 à 11,6 % en poids |
| CaO | 0 à 7,3 % en poids |
| SrO | 0 à 12,8 % en poids |
| F | 0 à 2,2 % en poids. |

8. Vitrocéramique à apatite selon l'une quelconque des revendications 1 à 7, qui contient un ou plusieurs oxydes métalliques lui conférant une couleur ou de la fluorescence.

9. Vitrocéramique à apatite selon la revendication 8, dans laquelle le métal de l'oxyde métallique ou les métaux des oxydes métalliques sont choisis dans le groupe des éléments 3d et 4f du système périodique des éléments.

10. Vitrocéramique à apatite selon l'une quelconque des revendications 1 à 9, dans laquelle la proportion des cristaux d'apatite est d'au moins 5 % en poids.

11. Vitrocéramique à apatite selon l'une quelconque des revendications 1 à 10, dans laquelle les cristaux d'apatite sont aciculaires.

12. Vitrocéramique à apatite selon l'une quelconque des revendications 1 à 11, dans laquelle les cristaux d'apatite ont une longueur moyenne de 0,1 à 10 µm.

13. Vitrocéramique à apatite selon l'une quelconque des revendications 1 à 12, qui présente dans la plage de température de 100 à 500 °C un coefficient de dilatation thermique de 6.10⁻⁶ K⁻¹ à 15.10⁻⁶ K⁻¹.

14. Vitrocéramique à apatite selon l'une quelconque des revendications 1 à 13, qui présente une température de calcination de 830 °C à 1 150 °C.

15. Vitrocéramique à apatite selon l'une quelconque des revendications 1 à 14, qui présente une résistance chimique < 80 µg/cm².

16. Procédé pour la production d'une vitrocéramique à apatite selon l'une quelconque des revendications 1 à 15, dans lequel
(a) on mélange les composants requis pour la production de la vitrocéramique et on les fait fondre en un verre,
(b) on convertit la masse de verre fondue provenant de l'étape (a) en un produit granulé vitreux,
(c) on fragmente éventuellement le produit granulé vitreux en une poudre ayant une taille moyenne de grain de 1 à 500 µm (sur la base du nombre des particules), et
(d) on soumet ensuite le produit granulé vitreux provenant de l'étape (b) ou la poudre de verre provenant de l'étape (c) à un traitement thermique en une ou plusieurs étapes dans la plage de température de 700 à 1 200 °C pendant une durée de 30 minutes à 6 heures.

17. Procédé selon la revendication 16, dans lequel on soumet le produit granulé vitreux provenant de l'étape (b) ou la poudre de verre provenant de l'étape (c) à un traitement thermique dans la plage de température de 900 à 1 200 °C.

18. Procédé selon la revendication 16 ou 17, dans lequel on soumet dans l'étape d) le produit granulé vitreux provenant de l'étape (b) ou la poudre de verre provenant de l'étape (c) à un traitement thermique pendant une durée de 30 minutes à 3 heures.

19. Matériau multicomposant, qui contient comme premier composant au moins une vitrocéramique à apatite selon l'une quelconque des revendications 1 à 15 sous forme de poudre et comme deuxième composant au moins une autre poudre de verre et/ou de vitrocéramique.

20. Matériau selon la revendication 19, qui contient, comme deuxième composant, un verre au silicate de zinc-métal alcalin qui contient les composants suivants :
| | |
|---|---|
| SiO₂ | 52,0 à 63,5 % en poids |
| Me'[III]₂O₃ | 8,5 à 13,0 % en poids |
| K₂O | 0 à 20,5 % en poids |
| Na₂O | 1,5 à 20,0 % en poids |
| Li₂O | 0 à 5,0 % en poids |
| ZnO | 3,6 à 8,0 % en poids |
| Me'[II]O | 2,5 à 6,5 % en poids |
| TiO₂ + ZrO₂ | 0,5 à 6,0 % en poids |
| SnO₂ | 0 à 9,5 % en poids |
| P₂O₅ | 0 à 4,0 % en poids |
| F | 0 à 2,0 % en poids |
| CeO₂ | 0 à 3,0 % en poids, |
(a) Me'[III]₂O₃ étant constitué de 0 à 13 % en poids de Al₂O₃ et de 0 à 9,5 % en poids de La₂O₃ ; et
(b) Me'[II]O étant constitué de 0 à 3,5 % en poids de CaO, de 0 à 4,5 % en poids de BaO et de 0 à 5,0 % en poids de MgO.

21. Vitrocéramique mixte, susceptible d'être obtenue par mélange des composants d'un matériau selon l'une quelconque des revendications 19 à 20 et ensuite conversion en une vitrocéramique mixte.

22. Utilisation d'une vitrocéramique à apatite selon l'une quelconque des revendications 1 à 15 ou d'une vitrocéramique mixte selon la revendication 21 à titre de composant d'opacité, de matériau dentaire, pour la production d'un matériau dentaire ou l'élaboration d'une restauration dentaire.

23. Utilisation selon la revendication 22, dans laquelle le matériau dentaire est un matériau de revêtement ou un matériau de placage.

24. Utilisation selon la revendication 22, dans laquelle la restauration dentaire est une couronne, un bridge, une couronne partielle, un onlay (incrustation de surface), une dent artificielle, une construction sur moignon ou une facette.
